# EUROPEAN PATENT APPLICATION

(11) **EP 0 576 766 A1**
(43) Date of publication of application: **05.01.1994**
(21) Application number: 92610053.8
(22) Date of filing: 29.06.1992
(51) Int. Cl.: C07D 295/08, C07D 317/64, C07D 233/61, C07C 217/48, C07D 295/14, C07D 295/12, A61K 31/445, A61K 31/495, A61K 31/535, A61K 31/415, A61K 31/135

(54) **Propanolamine derivatives, their preparation and use**

(71) Applicant: NOVO NORDISK A/S, DK-2880 Bagsvaerd (DK)
(72) Inventor: Jakobsen, Palle, DK-3500 Vaerlose (DK); Kanstrup, Anders, DK-2830 Virum (DK); Lundbeck, Jane Marie, DK-2600 Glostrup (DK)

(57) **Abstract**

Compounds of formula I 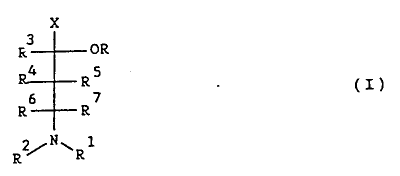
wherein
X is phenyl unsubstituted or optionally substituted with one or more cyano, halogeno, halogenoalkyl, C1-6-alkoxy, C1-6-alkyl, C1-5-alkanoyl, C3-5-alkenyl, aryloxy, aralkoxy, amino, C1-6-alkyl mono or disubstituted amino, C1-4-alkanoylamino, carbamoyl, C1-4-alkyl N-mono or disubstituted carbamoyl, C1-4-alkyl substituted with amino, C1-2-alkyl mono or disubstituted amino, N0₂, morpholino or imidazolyl; and
R is 3,4-methylenedioxyphenyl, aryl or heteroaryl all of which can be optionally substituted with one or more cyano, halogeno, C1-6-alkyl, C1-6-alkoxy, C2-6-alkenyl, trifluoromethyl, C3-5-alkylene, aryloxy, aralkoxy or C1-6-alkylthio; and
R¹ and R² are C1-10-alkyl, C3-7-cycloalkyl, C2-10-alkenyl, C3-6-cycloalkyl-C1-5-alkyl, all of which can be unsubstituted or substituted with C1-5-alkyl, C1-5-alkoxy or cyano; or
R¹ and R² together form a 5, 6 or 7 membered ring containing at least one nitrogen atom, or which optionally contains two nitrogen atoms, one or two oxygen atom(s) or one or two sulphur atom(s) or a combination thereof, which ring is unsubstituted or optionally substituted with C1-4-alkyl, C1-4-alkoxy or aryl; and
R³, R⁴, R⁵, R⁶, and R⁷ independently are hydrogen, C1-4-alkyl or phenyl; or
R⁴ and X together form a carbocyclic ring containing 5 or 6 atoms, or a salt thereof with a pharmaceutically acceptable acid; provided however that R¹ and R² are not C3-7-cycloalkyl, C1-10-alkyl or C2-10-alkenyl which may be straight, branched or cyclic, unsubstituted or substituted with C1-4-alkoxy, aryloxy or cycloalkyl, cyano or cycloalkylalkyl, when X is phenyl substituted with cyano, halogen, halogenalkyl, C1-6-alkoxy, C1-6-alkyl, C1-5-alkanoyl, C3-5-alkylene, aryloxy or aralkoxy, are useful in the treatment of anoxia, traumatic injury, ischemia, migraine, epilepsy, Parkinson's disease, Alzheimer's disease and other neurodegenerative diseases.

## Description

The present invention relates to therapeutically active aryloxyphenylpropylamines, a method of preparing the same and to pharmaceutical compositions comprising the compounds. The novel compounds are useful in the treatment of anoxia, traumatic injury, ischemia, migraine, epilepsy, Parkinson's disease, Alzheimer's disease and other neurodegenerative diseases.

It is well known that accumulation of calcium in the brain cells (calcium overload) is seen after periods of uncontrolled hyperactivity in the brain, such as after convulsions, migraine, anoxia and ischemia. As the concentration of calcium in the cells is of vital importance for the regulation of cell function, an uncontrolled high concentration of the cell calcium will lead to, or indirectly cause the symptoms and possibly also the degenerative changes combined with the above diseases.

Therefore, calcium overload blockers selective for brain cells will be useful in the treatment of anoxia, traumatic injury, ischemia, migraine, epilepsy, Parkinson's disease, Alzheimer's disease and other neurodegenerative diseases.

Well known calcium antagonists such as nifedipine, verapamil and diltiazem have activity against peripheral calcium uptake, e.g. in blood vessels and the heart, however, they have shown only very low activity against calcium overload in brain cells.

Accordingly it is an object of the invention to provide novel compounds having activity against calcium overload in brain cells.

The novel compounds of the invention are aryloxyphenylpropylamines of formula I 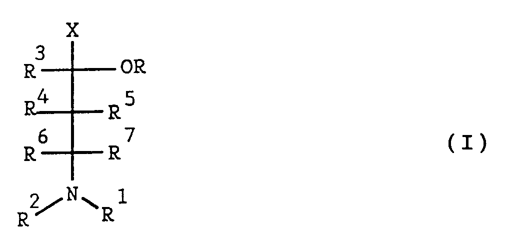
wherein
X is phenyl unsubstituted or optionally substituted with one or more cyano, halogeno, halogenoalkyl, C₁₋₆-alkoxy, C₁₋₆-alkyl, C₁₋₅-alkanoyl, C₃₋₅-alkenyl, aryloxy, aralkoxy, amino, C₁₋₆-alkyl mono or disubstituted amino, C₁₋₄-alkanoylamino, carbamoyl, C₁₋₄-alkyl N-mono or disubstituted carbamoyl, C₁₋₄-alkyl substituted with amino, C₁₋₂-alkyl mono or disubstituted amino, NO₂, morpholino or imidazolyl; and
R is 3,4-methylenedioxyphenyl, aryl or heteroaryl all of which can be optionally substituted with one ore more cyano, halogeno, C₁₋₆-alkyl, C₁₋₆-alkoxy, C₂₋₆-alkenyl, trifluoromethyl, C₃₋₅-alkylene, aryloxy, aralkoxy or C₁₋₆-alkylthio; and
R¹ and R² are C₁₋₁₀-alkyl, C₃₋₇-cycloalkyl, C₂₋₁₀-alkenyl, C₃₋₆-cycloalkyl-C₁₋₅-alkyl, all of which can be unsubstituted or substituted with C₁₋₅-alkyl C₁₋₅-alkoxy or cyano;
or
R¹ and R² together form a 5, 6 or 7 membered ring containing at least one nitrogen atom, or which optionally contains two nitrogen atoms, one or two oxygen atom(s) or one or two sulphur atom(s) or a combination thereof, which ring is unsubstituted or optionally substituted with C₁₋₄-alkyl, C₁₋₄-alkoxy or aryl; and
R³, R⁴, R⁵, R⁶ and R⁷ independently are hydrogen, C₁₋₄-alkyl or phenyl; or
R⁴ and X together form a carbocyclic ring containing 5 or 6 atoms, or a salt thereof with a pharmaceutically acceptable acid;
provided however that R¹ and R² are not C₃₋₇-cycloalkyl, C₁₋₁₀-alkyl or C₂₋₁₀-alkenyl which may be straight, branched or cyclic, unsubstituted or substituted with C₁₋₄-alkoxy, aryloxy or cycloalkyl, cyano or cycloalkylalkyl, when X is phenyl substituted with cyano, halogen, halogenalkyl, C₁₋₆-alkoxy, C₁₋₆-alkyl, C₁₋₅-alkanoyl, C₃₋₅-alkylene, aryloxy or aralkoxy.

Alkyl is intended to mean straight or branched alkyl radicals.

Examples of such salts include inorganic and organic acid addition salts such as hydrochloride, hydrobromide, sulphate, phosphate, acetate, fumarate, maleate, citrate, lactate, tartrate, oxalate, or similar pharmaceutically acceptable inorganic or organic acid addition salts.

The invention also relates to a method of preparing the above mentioned compounds. These methods comprise
a) reacting a compound of formula II 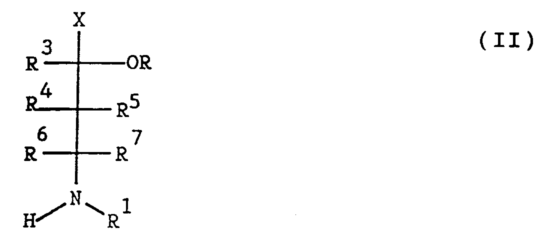 wherein X, R¹, R⁴, R⁵, R⁶, R⁷ and R have the meanings defined above, with a compound of formula R²-Y, wherein Y is a leaving group such as halogen and R² has the meaning defined above; or
b) reacting a compound of formula III 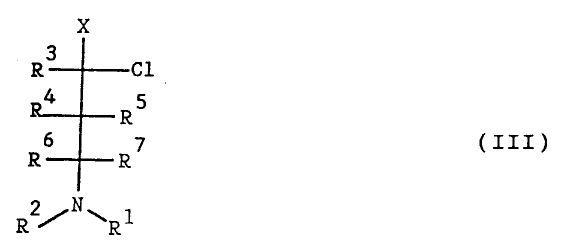 with a compound of formula IV

   ROH (IV)

   wherein R, R¹, R², R³, R⁴, R⁵, R⁶ and R⁷ have the meanings defined above; or
c) preparing a compound of formula III from the corresponding hydroxy compound by means of SOCl₂, where the hydroxy compound is prepared by a NaBH₄ reduction of the corresponding oxo-compound, which again is prepared by a Mannich reaction; or
d) preparing a compound of formula II by demethylating a compound of formula I by means of ROCOCl, especially R as -CHClCH₃, vinyl and -CH₂CCl₃ are preferable; or
e) reacting a compound of formula VI 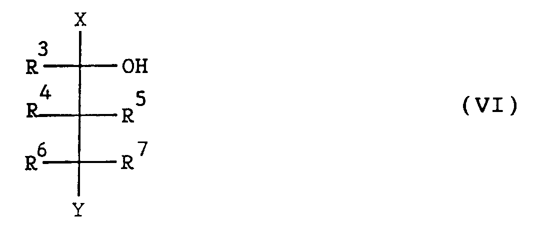 with a compound of formula R¹NHR² wherein X, R¹, R², R³, R⁴, R⁵, R⁶ and R⁷ have the meanings defined above, and Y is a leaving group such as halogen; or
f) reacting a compound of formula V 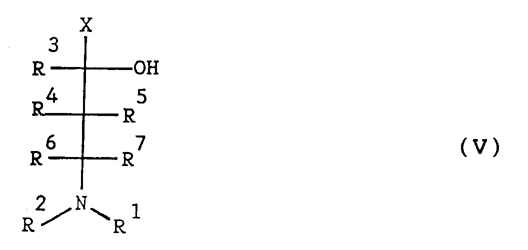 with a compound of formula 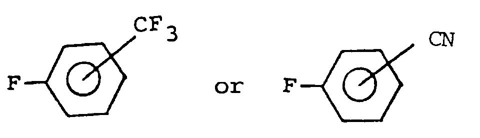 wherein X, R¹, R², R³, R⁴, R⁵, R⁶ and R⁷ have
   the meanings defined above; or
g) reacting a compound of formula V 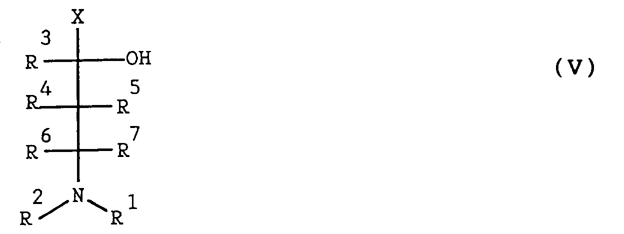 with a compound of formula IV

   ROH (IV)

   by means of Ph₃P and (EtOCON)₂ (Mitsunobu, O. Synthesis 1981 p.1); or
h) preparation of a compound of formula I by modification of the substituent in the X-group of a compound of formula I by known chemical methods.

These methods comprise reduction of NO₂-groups, alkylation of amino groups, hydrolysis of CN-groups giving acids or amides, N-alkylation of carboxamides, aromatic substitution of halogeno compounds or sulfonylation.

The pharmacological properties of the compounds of the invention can be illustrated by determining their capability to inhibit calcium uptake into brain synaptosomes.

### PRINCIPLE

Depolarization of neuronal membranes leads to an opening of socalled "voltage operated calcium channels" (VOC) in the membranes which allows a massive influx of calcium from the extracellular space. A crude synaptosomal preparation (socalled P₂ fraction) contains small vesicles surrounded by neuronal membrane and it is possible in such a preparation to study a depolarization-induced opening of VOC. In the present model ⁴⁵Ca influx is induced in the synaptosomes by depolarization with elevated potassium concentrations, and the effect of test substances on this stimulated uptake is studied (Nachshen, D.A. and Blaustein, M.P., Mol. Pharmcol., 16, 579 (1979)).

### ASSAY

A male Wistar rat is decapitated and the cerebral cortex removed and homogenized in 10 ml. of ice-cold 0.32 M sucrose using a glass homogenizer with a teflon pestle. All subsequent steps for isolation of synaptosomes are done at 0-4°C. The homogenate is centrifuged at 1000 x g for 10 min and the resulting supernatant is re-centrifuged at 18000 x g for 20 min. This pellet (P₂) is resuspended in 0.32 M sucrose (5 ml per g of original tissue) with a teflon pestle.

Aliquots (0.050 ml) of this crude synaptosomal suspension are added to glass tubes containing 0.625 ml of NaCl buffer (136 mM NaCl, 4 mM KCl, 0.35 mM CaCl₂, 1.2 mM MgCl₂, 20 mM Tris HCl, 12 mM glucose, pH 7.4) and 0.025 ml of various drug solutions in 48% Ethanol. The tubes are pre-incubated for 30 min on ice and then for 6 min at 37°C in a water bath.

The uptake is immediately initiated by adding 0.4 ml of ⁴⁵CaCl₂ (specific activity = 29-39 Ci/g; 0.5 µCi/assay), in 145 mM NaCl for non-depolarized samples and in 145 mM KCl for depolarized samples. The incubation is continued for 15 s.

The uptake is terminated by rapid filtration through GF-C glass fiber filters which are washed three times with 5 ml of a cold solution containing 145 mM KCl, 7 mM EGTA and 20 mM Tris HCl, pH 7.4. The amount of radioactivity on the filter disc is determined by liquid scintillation spectrometry.

### TEST PROCEDURE

Test substances are dissolved in 10 ml of 48% ethanol at a concentration of 0.44 mg/ml. Dilution are made in 48% ethanol to give final concentrations of 0.1, 0.3, 1, 3 and 10 µg/ml. Experiments are performed in triplicate. Controls for depolarized and nondepolarized samples are included in the assay and test substances are only tested in depolarized samples. 25-75% inhibition of stimulated uptake must be obtained before calculating the IC₅₀ value.

### RESULT

The test value will be given as IC₅₀ (the concentration (µg/ml) of test substance which inhibit 50% of stimulated uptake of ⁴⁵Ca (uptake in depolarized samples corrected for basal uptake in nondepolarized samples )). The IC₅₀ value is estimated from dose response curves.

Test results obtained by testing some compounds of the present invention will appear from the following table 1

**TABLE 1**

| Compound | IC₅₀ (µg/ml) |
|---|---|
| 2 | 16 |
| 3 | 17 |
| 5 | 7.5 |
| 15 | 6.1 |
| 17 | 4.9 |
| 20 | 8.0 |
| 23 | 4.0 |
| 24 | 5.3 |
| 25 | 2.2 |
| * Nifedipine | 26 |
| * Verapamil | 16 |
| * Diltiazem | > 90 |
| * Flunarizine | 20 |

| | |
|---|---|
| * well known calcium antagonists. | |

The compounds of the invention, together with a conventional adjuvant, carrier, or diluent, and if desired in the form of a pharmaceutically acceptable acid addition salt thereof, may be placed into the form of pharmaceutical compositions and unit dosages thereof, and in such form may be employed as solids, such as tablets of filled capsules, or liquids, such as solutions, suspensions, emulsions, elixirs, or capsules filled with the same, all for oral use, in the form of suppositories for rectal administration; or in the form of sterile injectable solutions for parenteral use (including subcutaneous administration and infusion). Such pharmaceutical compositions and unit dosage forms thereof may comprise conventional ingredients in conventional proportions, with or without additional active compounds or principles, and such unit dosage forms may contain any suitable effective calcium overload blocking amount of the active ingredient commensurate with the intended daily dosage range to be employed. Tablets containing ten (10) milligrams of active ingredient or, more broadly, ten (10) to hundred (100) milligrams, per tablet, are accordingly suitable representative unit dosage forms.

The compounds of this invention can thus be used for the formulation of pharmaceutical preparations, e.g. for oral and parenteral administration to mammals including humans, in accordance with conventional methods of galenic pharmacy.

Conventional excipients are such pharmaceutically acceptable organic or inorganic carrier substances suitable for parenteral or enteral application which do not deleteriously react with the active compounds.

Examples of such carriers are water, salt solutions, alcohols, polyethylene glycols, polyhydroxyethoxylated castor oil, gelatine, lactose, amylose, magnesium stearate, talc, silicic acid, fatty acid monoglycerides and diglycerides, pentaerythritol fatty acid esters, hydroxymethylcellulose and polyvinylpyrrolidone.

The pharmaceutical preparations can be sterilized and mixed, if desired, with auxiliary agents, emulsifiers, salt for influencing osmotic pressure, buffers and/or coloring substances and the like, which do not deleteriously react with the active compounds.

For parenteral application, particularly suitable are injectable solutions or suspensions, preferably aqueous solutions with the active compound dissolved in polyhydroxylated castor oil.

Ampoules are convenient unit dosage forms.

Tablets, dragees, or capsules having talc and/or a carbohydrate carrier or binder or the like, the carrier preferably being lactose and/or corn starch and/or potato starch, are particularly suitable for oral application. A syrup, elixir of the like can be used in cases where a sweetened vehicle can be employed.

Generally, the compounds of this invention are dispensed in unit form comprising 0.05-100 mg in a pharmaceutically acceptable carrier per unit dosage.

The dosage of the compounds according to this invention is 0.1-300 mg/day, preferably 10-100 mg/day, when administered to patients, e.g. humans, as a drug.

A typical tablet which may be prepared by conventional tabletting techniques contains:

| | |
|---|---|
| Active compound | 5.0 mg |
| Lactosum | 67.8 mg Ph.Eur. |
| Avicel™ | 31.4 mg |
| Amberlite™IRP 88 | 1.0 mg |
| Magnesii stearas | 0.25 mg Ph.Eur. |

Due to the high calcium overload blocking activity, the compounds of the invention are extremely useful in the treatment symptoms related to an accumulation of calcium in brain cells of mammals, when administered in an amount effective for blocking activity of compounds of the invention includes both activity against anoxia, traumatic injury, ischemia, migraine, epilepsy, Parkinson's disease, Alzheimer's disease and other neurodegenerative diseases. The compounds of the invention may accordingly be administered to a subject, e.g., a living animal body, including a human, in need of a calcium overload blocker, and if desired in the form of a pharmaceutically acceptable acid addition salt thereof (such as the hydrobromide, hydrochloride, or sulfate, in any event prepared in the usual or conventional manner, e.g., evaporation to dryness of the free base in solution together with the acid), ordinarily concurrently, simultanously, or together with a pharmaceutically acceptable carrier or diluent, especially and preferably in the form of a pharmaceutical composition thereof, whether by oral, rectal, or parenteral (including subcutanous) route, in an effective calcium overload blocking amount, and in any event an amount which is effective for the treatment of anoxia, traumatic injury, ischemia, migraine, epilepsy, Parkinson's disease, Alzheimer's disease and other neurodegenerative diseases due to their calcium overload blocking activity. Suitable dosage ranges are 1-200 milligrams daily, 10-100 milligrams daily, and especially 30-70 milligrams daily, depending as usual upon the exact mode of administration, form in which administered, the indication toward which the administration is directed, the subject involved and the body weight of the subject involved, and the preference and experience of the physician or veterinarian in charge.

The invention will now be described in further detail with reference to the following examples.

### EXAMPLE 1

### 3-Morpholino-1-phenyl-propan-1-ol (1)

Prepared from 3-iodo-1-phenyl-1-propanol (6.9 g) and morpholine (4.58 ml) by stirring at RT in THF (50 ml). Subsequent evaporation followed by ether/NaOH extraction and purification on a silica gel column using ether as eluent gave (1) (1.7 g) as a yellow oil, identified by ¹H and ¹³C NMR.

### 4-(3-(5-Indanyloxy)-3-phenylpropyl)morpholine, hydrochloride (2)

(1) (0.86 g), 5-indanol (0.54 g), triphenylphosphine (1.01 g) and diethylazodicarboxylate (0.61 ml) were reacted in dry THF (50 ml) under N₂-atmosphere in a Mitsunobu reaction. After stirring for 48 h at RT the mixture was evaporated, extracted with 4M NaOH/ether and subsequently the etheral layer was purified on silica gel with CH₂Cl₂/CH₃0H (9/1) as eluent. The resulting oil was precipitated with concentrated HCl from acetone solution giving 0.43 g colourless crystals. M.p. 214.2-214.4^{o}C.

### 4-(3-Phenyl-3-(4-trifluoromethylphenoxy)propyl)morpholine, oxalate (3)

(1) (0.86 g) and 4-fluorobenzotrifluoride (0.70 g) were dissolved in dry DMF (50 ml). After cooling to 0^{o}C potassium tert.butoxide (0.48 g) was added and the mixture was stirred overnight at RT. Subsequently the mixture was extracted with H₂O/ether, and the combined ether layers were evaporated. The resulting oil was purified on a silica gel column using ether as eluent and subsequently with pentane/triethylamine (1/1) as eluent. The latter purification was repeated three times resulting in an oil which was precipitated as the oxalate from acetone solution. Yield 0.51 g, m.p. 118.2-119.2^{o}C.

### EXAMPLE 2

### 3-(4-Methyl-1-piperazinyl)-1-phenyl-1-propanol (4)

3-Iodo-1-phenylpropan-1-ol (3 g) and N-methyl piperazine (6.35 ml) were stirred in DMSO (50 ml) for 14 days. The mixture was extracted several times with NaOH (4M) and ether. The combined etheral layers were evaporated and purified on silica gel using ether, followed by CH₃OH/CH₂Cl₂ (1/1) as eluent. Compound 4 was isolated as an oil (0.8 g) identified by ¹H and ¹³C NMR.

### 1-(3-Phenyl-3-(4-trifluoromethylphenoxy)propyl)-4-methyl piperazine, oxalate (5)

(4) (0.8 g) and 4-trifluoromethylphenol (0.55 g) were reacted under Mitsunobu conditions as described for (2). 0.73 g oil was isolated after the silica gel rinse up, resulting in 0.63 g after precipitation as the oxalate. M.p. 215.7-215.8^{o}C.

### 3-Iodo-1-phenyl-1-(4-trifluoromethylphenoxy)propane (6)

3-Iodo-1-phenyl-1-propanol (1 g) and 4-trifluoromethylphenol (0.62 g) reacted as described for (2) giving 0.7 g oil after purification on silica gel with ethyl acetate / triethylamine (10/1) as eluent. This oil was used without further purification.

### 1-(3-Phenyl-3-(4-trifluoromethylphenoxy)propyl)-4-(2-hydroxyethyl) piperazine (7)

0.7 g (6) and N-(2-hydroxyethyl) piperazine (1.12 g) were stirred in DMSO (50 ml) at RT for 7 days. The mixture was extracted several times with NaOH/ether, the combined etheral layers evaporated and purified on silica gel resulting in 0.15 g of (7) after precipitation as the oxalate, m.p. 199.7^{o}C.

### 3-(4-Phenyl-1-piperazinyl)-1-phenyl-propan-1-ol (8)

Prepared from 3-iodo-1-phenylpropan-1-ol (3 g) and N-phenyl piperazine (9.32 g) in DMSO (50 ml) as described for (4), resulting in 2.6 g (8) identified by ¹H and ¹³C NMR.

### 1-(3-Phenyl-3-(4-trifluoromethylphenoxy)propyl)-4-phenyl piperazine, oxalate (9)

Preparation from (8) (0.9 g) and 4-trifluoromethylphenol (0.49 g) as described for (2) gave 0.54 g after rinse up on silica gel. Precipitation as the oxalate gave 0.43 g (9), m.p. 161.5-162.7^{o}C.

### 1-(3-(5-Indanyloxy)-3-phenylpropyl)-4-phenylpiperazine, oxalate (10)

Preparation from (8) (0.8 g) and 5-indanol (0.36 g) as described for (2) gave 0.5 g (10) after precipitation. M.p. 169-170^{o}C.

### 1-(3-(3,4-Methylenedioxyphenoxy)-3-phenylpropyl)-4-phenylpiperazine, oxalate (11)

Preparation from (8) (0.9 g) and sesamol (0.42 g) as described for (2) gave 0.55 g (11) as the oxalate, m.p. 147.2-149.2^{o}C.

### EXAMPLE 3

### 3-Dimethylamino-1-(4-(1-imidazolyl)phenyl)-1-propanol (12)

Prepared from the Mannich product resulting from reaction between 1-acetyl-4-(1-imidazolyl)benzene (25 g), paraformaldehyde (5.24 g) and dimethylammonium chloride (14.2 g). 7.75 g of the resulting Mannich product was reduced with NaBH₄ (0.5 g) in ethanol resulting in 5.8 g (12) isolated as a yellow oil.

### N,N-dimethyl-3-(4-(1-imidazolyl)phenyl)-3-(4-trifluoromethylphenoxy)propanamine, hydrochloride (13)

(12) (2 g) was reacted with 4-fluorobenzotrifluoride (2 g) and potassium tert.butoxide (1.45 g) in dry DMF (50 ml) under N₂ as described for (3). The resulting yellow oil (3 g) was precipitated as the hydrochloride from acetone, m.p. 182.4 - 183^{o}C.

### N-Butyl-N-cyclopropylmethyl-3-(4-phenylphenyl)-3-(4-trifluoromethylphenoxy)propanamine, oxalate (14)

3-(N-butyl-N-cyclopropylamino)-1-(4-phenylphenyl)-1-propanol was prepared through a reaction sequence as described for compound (12) using 4-acetylbiphenyl, butylcyclopropylmethylammonium chloride, paraformaldehyde and NaBH₄ as reagents. 2 g of this alcohol was treated with 4-fluorobenzotrifluoride (1.17 g) and potassium tert butoxide (0.8 g) as described for (3). Purification on silica gel with ethyl acetate as eluent gave 3.4 g yellow oil which was precipitated as the oxalate, m.p. 93-98.4^{o}C.

### N,N-dimethyl-3-(4-nitrophenyl)-3-(3-trifluoromethylphenoxy)propanamine, hydrochloride (15)

3-Dimethylamino-1-(4-nitrophenyl)-1-propanol (16) was prepared through the normal Mannich reaction/reduction procedure. 2 g of (16) and 1.67 g of 3-trifluoromethylphenol were reacted as described for (2). After rinse up on silica gel 1.8 g of (15) was isolated which was precipitated as the hydrochloride, m.p. 170.1 - 172.8^{o}C.

### N,N-dimethyl-3-(4-nitrophenyl)-3-(5,6,7,8-tetrahydro-2-naphthoxy)propanamine, hydrochloride (17)

(16) (2 g) and 5,6,7,8-tetrahydro-2-naphthol (1.53 g) were reacted as described for (2). After purification two times on silica gel with CH₂Cl₂/CH₃OH (9/1) as eluent 2.2 g oil was precipitated as the hydrochloride by means of conc. HCl. M.p. 180.5 - 181.7^{o}C.

### N,N-dimethyl-3-(4-morpholinophenyl)-3-(4-trifluoromethylphenoxy)propanamine, oxalate (18)

3-Dimethylamino-1-(4-morpholinophenyl)-1-propanol was prepared from 4-morpholinoacetophenone, dimethyl ammonium chloride, paraformaldehyde and NaBH₄ through the reaction sequenced described for (12). 2.5 g of this alcohol was treated with 4-fluorobenzotrifluoride (1.25 ml) and potassium tert.butoxide (1.2 g) in DMF (50 ml) under N₂, as described for (3). Stirring at RT overnight. After purification 2.0 g oil was isolated. The oxalate was precipitated and recrystallized from acetone, m.p. 97-98^{o}C.

### EXAMPLE 4

### trans-2-(Piperidinomethyl)indan-1-ol (19)

Prepared from the Mannich product resulting from reaction between 1-indanone (10 g), paraformaldehyde (3.7 g) and piperidine hydrochloride (12 g) in refluxing ethanol. The crude Mannich base (19 g) obtained by evaporation of solvent followed by wash with ether was reduced by means of NaBH₄ (6.3 g) in methanol by stirring overnight at RT. The reaction mixture was filtered, evaporated to dryness and the resulting crude mass was extracted from OH⁻ with ether resulting in 7 g (19). The compound was identified by ¹H and ¹³C NMR which showed that it was contaminated with 13% of the cis-isomer. The crude mixture was used for further reaction without purification.

### trans-1-(1-(4-Trifluoromethylphenoxy)indan-2-yl)methyl piperidine, hydrochloride (20)

(19) (9 g) and 4-fluorobenzotrifluoride (7 g) were dissolved in dry DMF (200 ml). This mixture was treated with potassium tert. butoxide (4.8 g) and the reaction mixture rinsed up as described for compound (3). 7.6 g (29) m.p. 209-212^{o}C was isolated.

### trans-2-Piperidinomethyl-1-(4-trifluoromethylphenoxy)-1,2,3,4-tetrahydronaphthalene, hydrochloride (21)

This compound was prepared using the same reaction sequence as described for (19) and (2).

α-Tetralone (10 g), paraformaldehyde (2.8 g) and piperidine hydrochloride (9.1 g) gave 17.5 g crude Mannich base which on reduction with NaBH₄ (4.7 g) resulted in 10.3 g trans alcohol with no cis-isomer contamination as judged from the ¹H NMR spectrum. Further reaction of 10 g of this alcohol with 4-fluorobenzotrifluoride (6.4 g) and potassium tert. butoxide (4.5 g) gave 8.2 g of (21) m.p. 195-200^{o}C.

### EXAMPLE 5

### 1-(2,2-Dimethyl-3-phenyl-3-(4-trifluoromethylphenoxy)propyl)piperidine, hydrochloride (22)

Isobututyrophenone (10 g), paraformaldehyde (2.8 g) and piperidine hydrochloride (10 g) were refluxed in 1,2-dimethoxyethane. The crude reaction product (23.2 g) was isolated as described for (19) and reduced by means of NaBH₄ (7 g) in methanol resulting in 3.6 g alcohol after rinse up as for (19). This alcohol was treated with 4-fluorobenzotrifluoride (2.6 g) and potassium tert. butoxide (1.8 g) as described for (3) resulting in 2.2 g (22) m.p. 175^{o}C.

### EXAMPLE 6

### 1-(3-(4-Acetamidophenyl)-3-(4-trifluoromethylphenoxy)propyl)piperidine, oxalate (23)

4-Acetamidoacetophenone (10 g), paraformaldehyde (2.8 g), piperidine (9.3 ml) and conc. HCl (7.8 ml) were refluxed in 1,2-dimethoxyethane (100 ml) for 2 days. The mixture was evaporated and the residue heated with NaOH (4 M) and ether followed by CH₂Cl₂. HPLC showed that the ether phase contained 74% Mannich product and the CH₂Cl₂ phase 80% Mannich product. The CH₂Cl₂ phase was evaporated to dryness yielding 9.7 g crude product, which was purified on silica gel. 2.2 g of isolated Mannich product was treated with NaBH₄ (0.75 g) in ethanol (50 ml) and the mixture rinsed up as described for (19) yielding 0.47 g alcohol.

This was treated with 4-trifluoromethylphenol (0.28 g) under Mitsunobu conditions as described for (2). The crude product was treated with ether resulting in precipitation of triphenylphosphine oxide. The ether phase was evaporated, purified on silica gel with CH₂Cl₂/CH₃OH 9/1 as eluent resulting in 0.29 g (23) which was precipitated as the oxalate. M.p. 209-212^{o}C.

### 1-(3-(3-Acetamidophenyl)-3-(4-trifluoromethylphenoxy)propyl)piperidine, oxalate (24)

3-Acetamidoacetophenone (10 g), paraformaldehyde (2.8 g), piperidine (9.3 ml) and conc. HCl (7.8 ml) were reacted as described for (23), resulting in 4.1 g of the Mannich product which on reduction with NaBH₄ (1 g) gave 2.1 g alcohol which in turn was reacted with 4-trifluoromethylphenol (1.2 g), triphenylphosphine (1.95 g) and DEAD (1.25 g) giving 1 g of (24) after purification on silica gel as described for (23). M.p. 159-161^{o}C.

### 1-(3-(4-Cyanophenyl)-3-(4-trifluoromethylphenoxy)propyl)piperidine, oxalate (25)

Preparation from 1-(4-cyanophenyl)-3-piperidino-propan-1-ol (9.2 g) (26), 4-trifluoromethylbenzotrifluoride (6.8 g) and potassium tert. butoxide (4.6 g) in dry DMF (200 ml) as described for (20) gave 5.0 g (25). M.p. 190-193^{o}C. (26) was prepared from 4-acetylbenzonitrile through the usual Mannich reaction/reduction procedure.

### 1-(3-(4-Carbamoylphenyl)-3-(4-trifluoromethylphenoxy)propyl)piperidine, oxalate (27)

(25) (0.8 g) was treated with 0.7 g of a KF-Al₂O₃ mixture (prepared by mixing KF (4 g) and Al₂O₃ (W 200 neutral) (6 g) in water (60 ml) followed by evaporation to dryness) in tert. butanol (25 ml). After reflux for 4 h followed by cooling to RT and filtration, the filtrate was evaporated to dryness yielding 0.74 g reddish oil which was precipitated as the oxalate. M.p. 183-184^{o}C.

### 1-(3-(2-Chlorophenyl)-3-(4-trifluoromethylphenoxy)propyl)piperidine, oxalate (28)

Preparation from 1-(2-chlorophenyl)-3-piperidino-propan-1-ol (1.6 g), DEAD (1 g), triphenylphosphine (1.6 g) and 4-trifluoromethylphenol (1 g) as described for (2). After rinse up on silica gel 0.66 g yellow oil was isolated which after precipitation with oxalic acid in acetone gave 0.15 g (28). M.p. 170-171^{o}C.

### 1-(3-(4-Bromophenyl)-3-(4-trifluoromethylphenoxy)propyl)piperidine oxalate, (29)

1-(4-Bromophenyl)-3-piperidinopropan-1-ol (2.2 g) was treated with 4-fluorobenzotrifluoride (1.33 g) and potassium tert. butoxide (0.9 g) in dry DMF (100 ml) as described for (20). The rinse up gave a yellow oil which was precipitated as the oxalate. Yield 1.2 g. M.p. 176-177^{o}C.

### EXAMPLE 7

### threo-1-(2-Methyl-3-phenyl-3-(4-trifluoromethylphenoxy)propyl)piperidine (30)

2-Methyl-1-phenyl-3-piperidinopropan-1-ol (5 g) (a mixture of the erythro and threo isomers (ratio erythro/threo 1/3)) was treated with 4-fluorobenzotrifluoride (3.88 g) and potassium tert. butoxide (2.65 g) as described for (20). Purification on silica gel column with CH₂Cl₂/CH₃OH 19/1 as eluent gave (2 g) of the threo form which was precipitated as the oxalate. M.p. 182-184^{o}C. The actual stereoisomeric form was estimated from the ¹H NMR spectra.

## Claims

1. A compound of formula I 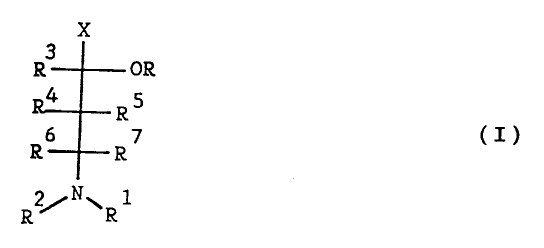 wherein
X is phenyl unsubstituted or optionally substituted with one or more cyano, halogeno, halogenoalkyl, C₁₋₆-alkoxy, C₁₋₆-alkyl, C₁₋₅-alkanoyl, C₃₋₅-alkenyl, aryloxy, aralkoxy, amino, C₁₋₆-alkyl mono or disubstituted amino, C₁₋₄-alkanoylamino, carbamoyl, C₁₋₄-alkyl N-mono or disubstituted carbamoyl, C₁₋₄-alkyl substituted with amino, C₁₋₂-alkyl mono or disubstituted amino, NO₂, morpholino or imidazolyl; and
R is 3,4-methylenedioxyphenyl, aryl or heteroaryl all of which can be optionally substituted with one ore more cyano, halogeno, C₁₋₆-alkyl, C₁₋₆-alkoxy, C₂₋₆-alkenyl, trifluoromethyl, C₃₋₅-alkylene, aryloxy, aralkoxy or C₁₋₆-alkylthio; and
R¹ and R² are C₁₋₁₀-alkyl, C₃₋₇-cycloalkyl, C₂₋₁₀-alkenyl, C₃₋₆-cycloalkyl-C₁₋₅-alkyl, all of which can be unsubstituted or substituted with C₁₋₅-alkyl, C₁₋₅-alkoxy or cyano;
or
R¹ and R² together form a 5, 6 or 7 membered ring containing at least one nitrogen atom, or which optionally contains two nitrogen atoms, one or two oxygen atom(s) or one or two sulphur atom(s) or a combination thereof, which ring is unsubstituted or optionally substituted with C₁₋₄-alkyl, C₁₋₄-alkoxy or aryl; and
R³, R⁴, R⁵, R⁶ and R⁷ independently are hydrogen, C₁₋₄-alkyl or phenyl; or
R⁴ and X together form a carbocyclic ring containing 5 or 6 atoms, or a salt thereof with a pharmaceutically acceptable acid;
provided however that R¹ and R² are not C₃₋₇-cycloalkyl, C₁₋₁₀-alkyl or C₂₋₁₀-alkenyl which may be straight, branched or cyclic, unsubstituted or substituted with C₁₋₄-alkoxy, aryloxy or cycloalkyl, cyano or cycloalkylalkyl, when X is phenyl substituted with cyano, halogen, halogenalkyl, C₁₋₆-alkoxy, C₁₋₆-alkyl, C₁₋₅-alkanoyl, C₃₋₅-alkylene, aryloxy or aralkoxy.

2. The compound according to claim 1 selected from
4-(3-(5-Indanyloxy)-3-phenylpropyl)morpholine,
4-(3-phenyl-3-(4-trifluoromethylphenoxy)propyl)morpholine,
1-(3-phenyl-3-(4-trifluoromethylphenoxy)propyl)-4-methyl piperazine,
1-(3-phenyl-3-(4-trifluoromethylphenoxy)propyl)-4-(2-hydroxyethyl) piperazine,
1-(3-phenyl-3-(4-trifluoromethylphenoxy)propyl)-4-phenyl piperazine,
1-(3-(5-indanyloxy)-3-phenylpropyl)-4-phenylpiperazine,
1-(3-(3,4-methylenedioxyphenoxy)-3-phenylpropyl)-4-phenyl piperazine,
N,N-dimethyl-3-(4-(1-imidazolyl)phenyl)-3-(4-trifluoromethylphenoxy)propanamine,
N-Butyl-N-cyclopropylmethyl-3-(4-phenylphenyl)-3-(4-trifluoromethylphenoxy)propanamine,
N,N-dimethyl-3-(4-nitrophenyl)-3-(3-trifluoromethylphenoxy)propanamine,
N,N-dimethyl-3-(4-nitrophenyl)-3-(5,6,7,8-tetrahydro-2-naphthoxy)propanamine,
N,N-dimethyl-3-(4-morpholinophenyl)-3-(4-trifluoromethylphenoxy)propanamine,
trans-1-(1-(4-Trifluoromethylphenoxy)indan-2-yl)methyl piperidine,
trans-2-Piperidinomethyl-1-(4-trifluoromethylphenoxy)-1,2,3,4-tetrahydronaphthalene,
1-(2,2-Dimethyl-3-phenyl-3-(4-trifluoromethylphenoxy)propyl)piperidine,
1-(3-(4-Acetamidophenyl)-3-(4-trifluoromethylphenoxy)propyl)piperidine,
1-(3-(3-Acetamidophenyl)-3-(4-trifluoromethylphenoxy)propyl)piperidine,
1-(3-(4-Cyanophenyl)-3-(4-trifluoromethylphenoxy)propyl)piperidine,
1-(3-(4-Carbamoylphenyl)-3-(4-trifluoromethylphenoxy)propyl)piperidine,
1-(3-(2-Chlorophenyl)-3-(4-trifluoromethylphenoxy)propyl)piperidine,
1-(3-(4-Bromophenyl)-3-(4-trifluoromethylphenoxy)propyl)piperidine,
1-(2-Methyl-3-phenyl-3-(4-trifluoromethylphenoxy)propyl)piperidine,
or a pharmaceutically acceptable salt thereof.

3. A method of preparing a compound according to claim 1 characterized in
a) reacting a compound of formula II 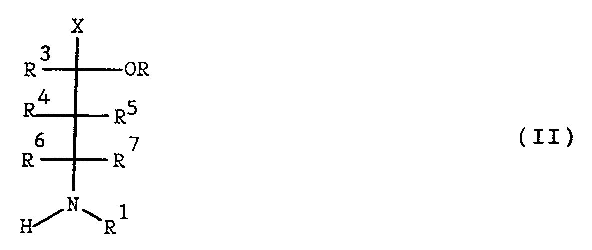 wherein X, R¹, R⁴, R⁵, R⁶, R⁷ and R have the meanings defined above, with a compound of formula R²-Y, wherein Y is a leaving group such as halogen and R² has the meaning defined above; or
b) reacting a compound of formula III 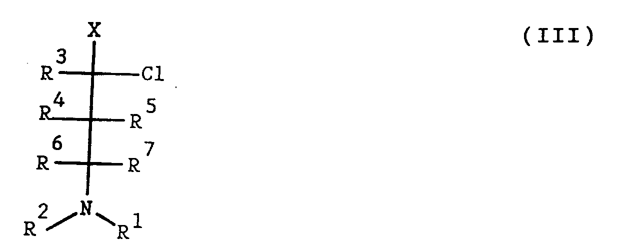 with a compound of formula IV
ROH (IV)
wherein R, R¹, R², R³, R⁴, R⁵, R⁶ and R⁷ have the meanings defined above; or
c) preparing a compound of formula III from the corresponding hydroxy compound by means of SOCl₂, where the hydroxy compound is prepared by a NaBH₄ reduction of the corresponding oxo-compound, which again is prepared by a Mannich reaction; or
d) preparing a compound of formula II by demethylating a compound of formula I by means of ROCOCl, especially R as -CHClCH₃, vinyl and -CH₂CCl₃ are preferable; or
e) reacting a compound of formula VI 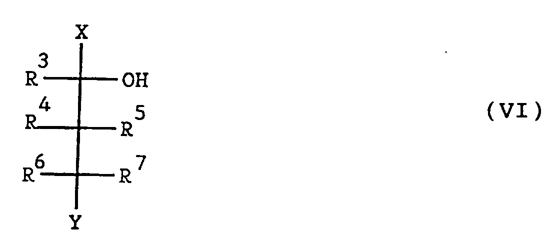 with a compound of formula R¹NHR² wherein X, R¹, R², R³, R⁴, R⁵, R⁶ and R⁷ have the meanings defined above, and Y is a leaving group such as halogen; or
f) reacting a compound of formula V 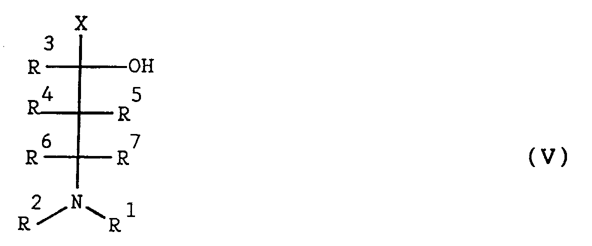 with a compound of formula 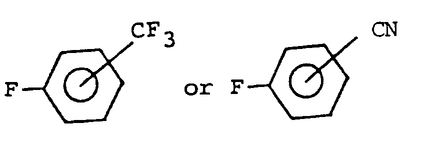 wherein X, R¹, R², R³, R⁴, R⁵, R⁶ and R⁷ have
the meanings defined above; or
g) reacting a compound of formula V 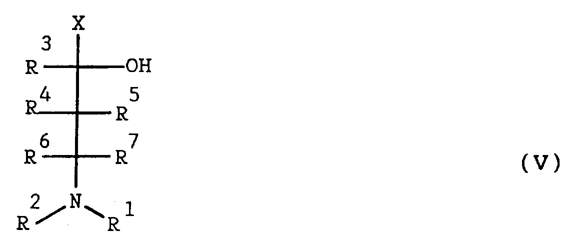 with a compound of formula IV
ROH (IV)
by means of Ph₃P and (EtOCON)₂; or
h) preparation of a compound of formula I by modification of the substituent in the X-group of a compound of formula I by known chemical methods.

4. A pharmaceutical composition comprising a compound of claim 1 or a salt thereof with a pharmaceutically acceptable acid together with a pharmaceutically acceptable carrier or diluent.

5. A pharmaceutical composition suitable for use in preventing calcium overload in brain cells of mammals, including humans, comprising an amount of a compound of claim 1, which is effective for inhibiting calcium uptake into brain cells together with a pharmaceutically acceptable carrier or diluent.

6. The pharmaceutical composition according to claim 4 or 5 wherein it is in the form of an oral dosage unit containing 1-100 mg of the active compound.

7. A method of treating calcium overload in brain cells of mammals, including humans, comprising administering a calcium overload blocking amount of a compound according to claim 1.

8. A method of treating calcium overload in brain cells of mammals, including humans, comprising administering a pharmaceutical composition according to claim 5.

9. The use of a compound according to claim 1 or a salt thereof with a pharmaceutically acceptable acid for the preparation of a medicament useful in treatment of calcium overload in brain cells of mammals, including humans.
